# EUROPEAN PATENT SPECIFICATION

(11) **EP 0 706 802 B1**
(45) Date of publication and mention of the grant of the patent: **24.03.2004**
(21) Application number: 95117751.8
(22) Date of filing: 27.07.1989
(51) Int. Cl.: A61L 2/18, A61K 9/08

(54) **A method of preserving ophthalmic solutions and compositions therefor**
Verfahren zur Konservierung ophthalmischer Lösungen und Zusammensetzungen dafür
Procédé de préservation de solutions ophthalmiques et des compositions à cet effet

(30) Priority: 04.08.1988 US 229163
(43) Date of publication of application: 17.04.1996
(62) Divisional of application: 89810578.8
(73) Proprietor: Novartis AG, 4056 Basel (CH); Novartis Pharma GmbH, 1230 Wien (AT)
(72) Inventor: Martin, Stephen M., Roswell, GA 30075 (US); Tsao, Fu-Pao, Lawrenceville, GA 30243 (US)

(56) References cited:
- EP-A- 0 009 839
- EP-A- 0 265 381
- EP-A- 0 289 463
- FR-A- 2 134 666

## Description

The present invention relates to a method of preserving ophthalmic solutions with trace amounts of stabilized peroxy compounds. More particularly, this invention relates to the use of stabilized trace amounts of hydrogen peroxide as preservative in buffered saline for eye care solutions.

Hydrogen peroxide is a well-known germicidal agent. For example, hydrogen peroxide in the form of a relatively dilute solution, e.g. about 0.5 to 6 % by weight in water, is known to be effective as a disinfectant for use with contact lenses in order to kill any contaminating microorganisms.

One drawback with unstabilized dilute hydrogen peroxide solutions, however, is that without the use of a stabilizer or a combination of stabilizers, the aqueous peroxide solutions characteristically decompose over a period of time. The rate at which such dilute hydrogen peroxide solutions decompose will, of course, be dependent upon such factors as pH and the presence of trace amounts of various metal impurities, such as copper or chromium, which may act to catalytically decompose the same. Moreover, at moderately elevated temperatures, the rate of decomposition of such dilute aqueous hydrogen peroxide solutions is greatly accelerated.

A large variety of stabilizers have been proposed for use with hydrogen peroxide to deactivate trace catalytic impurities, including stannous salts, ethylene diamine tetraacetic acid, and the like. For example, U.S. Patent No. 3,860,391 discloses bleaching compositions containing hydrogen peroxide and, as a stabilizer, amino lower alkylene phosphates, including diethylene triamine penta(methylene phosphonic acid) or salts thereof, and/or hydroxy alkane phosphates, with or without additional stabilizer constituents, and adjusted to a pH of between about 9.0 and 12.0 with, e.g. sodium hydroxide, for the bleaching of cellulosic materials. Exemplified are compositions having a pH of 12.0. However, such highly basic compositions are undesirable in ophthalmically-related solutions, including eyewashes and contact lens cleaning solutions.

Also, British Patent No. 1,500,707 discloses a contact lens sterilizing solution using hydrogen peroxide with 200-2000 ppm of a phosphate [pyrophosphate] stabilizer at a pH of 4.5.

However, the disinfection is not carried out at a pH consistent with the ocular environment since the pH needs to be elevated to around 7. Also, the peroxide must be eliminated so as to make the solution compatible with the eye.

U.S. Patent 4,304,762 discloses stabilization of aqueous hydrogen peroxide by addition of diethylenetriamine penta(methylene phosphonic acid) or a salt thereof at a pH of about 7. However, the stabilizing phosphonate compound is reported to be particularly effective for use with alkaline hydrogen peroxide solutions. Further these solutions are disclosed as being of use as a base for liquid bleach products. There is neither any hint that trace amounts of hydrogen peroxide are effectively stabilized nor is there any indication that hydrogen peroxide might be used as a preservative for an ophthalmic solution.

Some of the eye care solutions commercially available today use benzalkonium chloride, rather than hydrogen peroxide, as a preservative. For example, contact lens solutions typically contain 0.9 % sodium chloride, buffers, surfactants, wetting agents, and 0.002 to 0.01 % benzalkonium chloride. Benzalkonium chloride is also used in other products, including isotonic decongestant ophthalmic solutions, such as Visine® eyedrops manufactured by the Leeming Division of Pfizer, Inc.

A problem exists, however, in that benzalkonium chloride, being cationic in character, reacts with proteins found in the ocular environment and causes unwanted deposits on soft contact lenses. Benzalkonium chloride and its analogs are also taken up by lens material and can have a deleterious effect on the structure of the lens [Davis, S.S. et al., "The Adsorption of Cationic Antimicrobial Agent Onto PolyHema", Colloids and Surfaces, 12, 203-212 (1984)]. In addition, benzalkonium chloride is inactivated by many compounds, including those associated with cotton and nylon fibers. Furthermore, in Swan, K.C., "Reactivity of the Ocular Tissues to Wetting Agents", Am. J. Ophthalmol., 27, 118 (1944), it was noted that repeated use of benzalkonium chloride at concentrations of 1:5000 or stronger can denature the corneal protein and cause irreversable damage. It was also found that 0.04 % to 0.05 % solutions of benzalkonium chloride can cause superficial puncture disturbance of the corneal epithelium.

Other preservatives currently in use include thimerosal, which can cause a sensivity reaction to the eye, and sorbic acid, which commonly causes lens discoloration. The disadvantages of the commonly used preservatives such as thimerosal, benzalkonium, chloride and others are discussed in the following literature: M. Sibley, et al., "Understanding Sorbic Acid-Preserved Contact Lens Solutions", International Contact Lens Clinic 11 (9), 531 (1984); M. Orron et al., "Measurement of Preservative Binding with Soflens® (polymacon) Contact Lens", Aust. J. Optom. 59, 277 (1976); and M. Akers, "Considerations in selecting antimicrobial preservative agents for parenteral product development", Pharmaceutical Technology, May, p. 36 (1984).

Non-stabilized contact lens sterilization solutions comprising a peroxide compound optionally in combination with thimerosal are known from FR-A-2 134 666. The stabilization of 0.5 to 6 % H₂O₂ contact lens disinfection compositions with diethylene triamine penta(methylene-phosphonic acid) or with a combination of specific diphosphonic acid alkanols and a second peroxide stabilizer is known from EP-A-265 381 or EP-A-289 463.

An object of the invention is to provide preserved ophthalmic solutions with hydrogen peroxide generating components.

Surprisingly, the disadvantages of the prior art preservatives are overcome by stabilized trace peroxy compounds provided by the present invention which may be used as a preservative in ophthalmic solutions such as contact lens cleaning solutions.

The low levels of peroxy compounds are below the commercially suitable amounts necessary for the peroxy compound to act as disinfectant and is low enough to be tolerable to direct application to the eye. In addition, the pH is also compatible with the ocular environment.

The invention therefore relates to a preserved, buffered, isotonic, saline contact lens solution which is compatible with the ocular environment and has a pH of from 5.5 to 8, characterized in that said solution consists essentially of
(i) sodium perborate or sodium peroxide as a hydrogen peroxide source providing hydrogen peroxide in an amount of 0.001 % and more but less than 0.01 % (100 ppm) by weight,
(ii) a hydrogen peroxide stabilizer, which is a phosphonic acid selected from a compound of the formula (I) 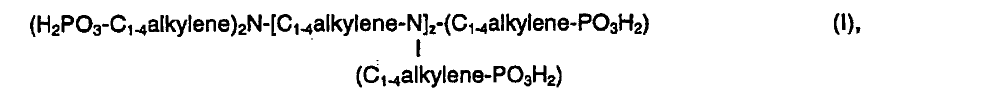 wherein z is an integer of from 0-3; and
   a compound of the formula (II) 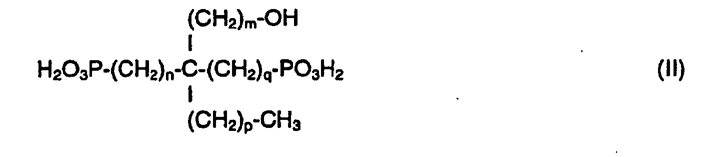 wherein each of n, m, p and q is independently 0-4,
   and physiologically compatible salts thereof,
(iii) a buffer and
(iv) water, sodium chloride and optionally a further tonicity enhancing agent.

For example, the trace amount hydrogen peroxide in these ophthalmic solutions is stabilized by about 0.002 % (20 ppm) to about 0.03 % (300 ppm) by weight of diethylene triamine penta(methylene phosphonic acid) or a physiologically compatible salt thereof.

Alternatively or additionally, from 0.005 % to about 0.2 % by weight of 1-hydroxy-ethylidene-1,1-diphosphonic acid can be added to the solution.

The source of hydrogen peroxide, which provides an effective resultant amount of hydrogen peroxide, includes sodium perborate decahydrate and sodium peroxide.

The solutions of the present invention are ophthalmic active agent free and may be used as preserved saline, as a preserved contact lens stabilizing solution or as a preserved contact lens care solution for contact lenses during non-wearing periods.

The peroxy stabilizers used in the present invention are
(a) compounds of the formula 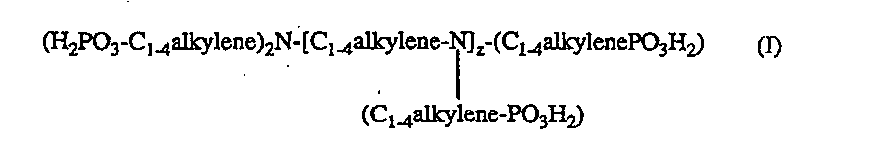 wherein z is an integer of from 0-3;
   and
(b) compounds of the formula 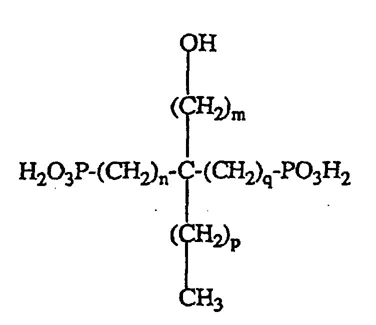
wherein each of n, m, p and q is independently 0-4,
or physiologically compatible salts thereof. Highly preferred within formula I are compounds wherein z is 2 and compounds wherein each C₁₋₄alkylene group is C₁ or C₂. Most preferred within formula I is diethylene triamine penta(methylene-phosphonic acid, and the physiologically compatible salts thereof, marketed by Monsanto under the name Dequest® 2060. Highly preferred within formula II are compounds wherein n, m, p and q are each 0 or 1, most preferably zero, or a physiologically compatible salt thereof, marketed by Monsanto under the name Dequest® 2010.

Physiologically compatible salts of the compounds of formulae I and II include, for example, water soluble salts with conventional pharmaceutically acceptable cationic moieties, including the alkali metal, e.g. sodium, potassium, alkaline earth metal, e.g. calcium, ammonium and amine cations. Suitable amine salts include, for example, mono-, di-, and tri-lower alkyl amines, e.g. methylamine, ethylamine, diethylamine, triethylamine, dimethylamine, trimethylamine, propylamine, and the like; and mono-, di-, and tri-lower hydroxyalkyl amines, e.g. ethanolamine, diethanolamine, triethanolamine, glucamine, 2-hydroxypropylamine, and the like. By "lower" in the context of an alkyl group is meant an alkyl group having up to 6 carbon atoms, preferably up to 4 carbon atoms.

Preferably, the concentration of the stabilizer of formula I or salt thereof is present in the stabilized composition in an amount between about 0.006 and about 0.02 % by weight of the composition, and most preferably between about 0.006 and about 0.0120 % by weight of the composition.

Even preferably the stabilizer of formula II is present per 100 g of solution in an amount of at least about 0.024 mmole (50 ppm), preferably 0.039 mmole (80 ppm) up to about 0.34 mmole (700 ppm) more preferably 0.049 mmole (100 ppm) up to about 0.29 mmole (600 ppm), most preferably 0.073 mmole (150 ppm) to about 0.19 mmole (400 ppm). The amounts in parentheses are for Dequest® 2010 which has a molecular weight of 206. Other stabilizers of formula II should be present in molar equivalents thereto.

Preferably the pH of the stabilized solution is between about 5.5 and about 8. More preferably, the pH of the stabilized hydrogen peroxide solution is between about 6.0 and 8.0, most preferable between about 6.5 and 7.5. The pH can be adjusted as desired by incorporation of suitable amounts of acid or base of a physiologically tolerable nature in the amounts employed, e.g. hydrochloric acid or e.g. sodium hydroxide.

The pH of the stabilized solution presents another advantage over the prior art since the pH's of most existing ophthalmic solutions containing hydrogen peroxide are relatively low. The pH values of available hydrogen peroxide products for contact lenses are listed as follows:

| Name of the Product | pH | % of H₂O₂ |
|---|---|---|
| AOSept (CIBA Vision) | 6.3-6.6 | 3.3-3.5 |
| Lensept (CIBA Vision) | 3.98 | 3.4 |
| Oxysept (Allergan) | 3.28 | 3.3 |
| Mirasept (Coopervision) | 3.96 | 3.6 |
| Quiksept (Bausch & Lomb) | 3.57 | 3.5 |
| Puresept (Abbott Labs) | 3.83 | 3.4 |
| Softmate II (Barnes Hind) | 3.5-3.6 | 3.5 |

Also, there may be present in the stabilized hydrogen peroxide solution according to the present invention one or more conventional, substantially inert, physiologically acceptable tonicity enhancing agents. Suitable such agents include, for example, alkali metal halides, phosphates, hydrogen phosphate, and borates. Preferred are sodium chloride, sodium phosphate monobasic and sodium phosphate dibasic. The function of such tonicity enhancing agents is to assure approximate physiologic tonicity to the solution which is instilled in the eye or to help assure such tonicity upon dilution if dilution is necessary prior to contact with the eye due to peroxide content as indicated above.

Preferably sufficient tonicity enhancing agents are present in the solution so that it is substantially isotonic or, such that, upon decomposition or dilution of the hydrogen peroxide therein, the resulting solution is substantially isotonic, e.g. substantially equivalent in tonicity to a 0.9 % by weight aqueous sodium chloride solution.

In general, the stabilized hydrogen peroxide solutions of the present invention are characterized by their extraordinary stability, even under accelerated conditions, for example by heating the solutions to 100°C for 24 hours. Thus, the shelf life of these compositions is enhanced. Moreover, the instant compositions are characterized by their physiological tolerability subsequent to hydrogen peroxide decomposition.

Another advantage in using hydrogen peroxide in ophthalmic solutions is that the trace amount of hydrogen peroxide, especially less than 100 ppm, is destroyed once it comes in contact with the eye. For example, catalase existing in the eye tissue will cause the breakdown of the hydrogen peroxide into water and oxygen. As a result, the solution, upon application, becomes preservative free and greatly minimizes adverse reactions. The problems associated with other preservatives, such as the inability to break down innocuous compounds, are eliminated.

Formulation of the solutions of the invention can be made in any conventional manner. For example, all of the components other than the peroxy compound and water can be placed in a container and peroxy compound added thereto with mixing. Alternatively the dry components can be rubbed up with a small portion of liquid stabilizer, then the remainder of the stabilizer added, followed by the peroxy compound, and most of the water. The viscosity enhancing agent, i.e. thickener, can then be added or the formed solution can be added to the thickener. One of ordinary skill in the art will be aware of numerous variations in the manner of formulating the solutions of the invention.

The following examples are presented for illustrative purposes and are not intended to limit the scope of this invention, but to demonstrate the stability of the peroxy solutions as stabilized in accordance with the present invention. All parts are by weight unless otherwise indicated.

Example 1: Dissolve 0.35 g of sodium chloride, 0.35 g of potassium chloride, 0.58 g boric acid, 0.005 g sodium borate decahydrate and 0.006 g diethylene triamine penta(methylene phosphonic acid) in 80 ml of deionized water. Add 0.0238 g sodium perborate. Add water up to 100 ml and adjust the pH to 7 by the addition dropwise of diluted hydrochloric acid or sodium hydroxide. The hot stability of this solution is above 91 %.

Example 2: The procedure of example 1 is duplicated except that the 0.35 g of potassium chloride are replaced by 0.35 g of calcium chloride. The hot stability of this solution is above 93 %.

Example 3: Dissolve 0.61 g of sodium chloride, 0.50 g boric acid, 0.005 g sodium borate decahydrate and 0.006 g diethylene triamine penta(methylene phosphonic acid) in 80 ml of deionized water. Add 0.0133 g sodium peroxide (from Mallinckrodt Cat. No. 7864). Add water up to 100 ml and adjust the pH to 7 by the addition dropwise of diluted hydrochloric acid or sodium hydroxide. The hot stability of this solution is above 93 %.

Comparative Example: The procedure of example 3 is duplicated except that the 0.0133 g sodium peroxide are replaced by 0.0138 g of urea hydrogen peroxide (from Aldrich Cat. No. 28913-2). The hot stability of this solution is above 53.6 %.

Example 4: Dissolve 0.61 g of sodium chloride, 0.50 g boric acid, 0.005 g sodium borate decahydrate, and various amounts of 1-hydroxyethylidene-1,1-diphosphonic acid in 80 ml of deionized water. Add 0.0238 g sodium perborate. Add water up to 100 ml and adjust the pH to 7 by the addition dropwise of diluted hydrochloric acid or sodium hydroxide. The hot stabilities of these solutions are listed as follows:

| 1-Hydroxyethylidene-1,1-diphosphonic acid (g) | Hot Stability % |
|---|---|
| 0.03 | 87.1 |
| 0.05 | 90.3 |
| 0.08 | 96.8 |

Example 5: Microbial preservative effectiveness test results of 50 ppm hydrogen peroxide in a borate buffer are listed as follows:

### PRESERVATIVE EFFECTIVENESS TEST (50 ppm H₂O₂ solution)

| Challenge Microbe | Original Inoculum | Day 7 | Day 14 | Rechallenge on Day 14 with Inoculum |
|---|---|---|---|---|
| E. coli | 1.67x10⁶ | Zero | Zero | 1.60x10⁵ |
| P. aeruginosa | 2.04x10⁶ | Zero | Zero | 1.94x10⁵ |
| S. aureus | 1.09x10⁶ | 1.75x10² | Zero | 1.74x10⁵ |
| A. niger | 1.46x10⁵ | 2.15x10⁴ | 8.40x10³ | 1.00x10⁵ |
| C. albicans No. 1 | 1.63x10⁶ | 8.00x10² | 2.40x10² | 1.41x10⁵ |
| C. albicans No. 2 | 1.53x10⁶ | 1.35x10³ | 3.40x10² | 1.41x10⁵ |
| C. albicans No. 3 | 1.63x10⁶ | 1.75x10³ | 4.90x10² | 1.41x10⁵ |

| Challenge Microbe | Day 21 | Day 28 | % Reduction | Pass/Fail |
|---|---|---|---|---|
| E. coli | Zero | Zero | 100 % | Pass |
| P. aeruginosa | Zero | Zero | 100 % | Pass |
| S. aureus | Zero | Zero | 100 % | Pass |
| A. niger | 4.65x10⁴ | 4.50x10⁴ | | Pass |
| C. albicans No. 1 | 1.09x10⁵ | 3.90x10⁴ | | Pass |
| C. albicans No. 2 | 1.12x10⁵ | 3.55x10⁴ | | Pass |
| C. albicans No. 3 | 1.26x10⁵ | 3.40x10⁴ | | Pass |

## Claims

1. A preserved, buffered, isotonic, saline contact lens solution which is compatible with the ocular environment and has a pH of from 5.5 to 8, **characterized in that** said solution consists essentially of
(i) sodium perborate or sodium peroxide as a hydrogen peroxide source providing hydrogen peroxide in an amount of 0.001 % and more but less than 0.01 % (100 ppm) by weight,
(ii) a hydrogen peroxide stabilizer, which is a phosphonic acid selected from
a compound of the formula (I) 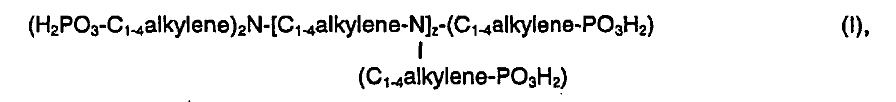 wherein z is an integer of from 0-3; and
a compound of the formula (II) 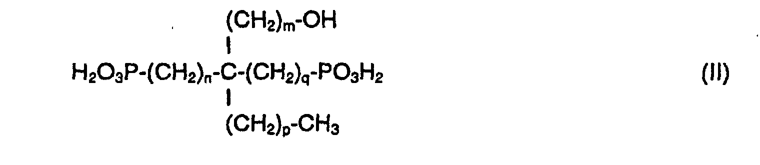 wherein each of n, m, p and q is independently 0-4,
and physiologically compatible salts thereof,
(iii) a buffer and
(iv) water, sodium chloride and optionally a further tonicity enhancing agent.

2. A solution according to claim 1, wherein said solution has a pH of between 6.5 to 7.5.

3. A solution according to claim 1 or 2, wherein sodium perborate is used as hydrogen peroxide source.

4. A solution according to claim 3, wherein a sodium containing borate compound and boric acid are used as buffer (iii).

5. A solution according to any one of claims 1 to 4, wherein said hydrogen peroxide stabilizer is diethylene triamine penta(methylene phosphonic acid) or a physiologically acceptable salt thereof.

6. A solution according to any one of claims 1 to 5 comprising from 0.002 % to 0.03 % hydrogen peroxide stabilizer.

7. A solution according to any one of claims 1 to 4, wherein said hydrogen peroxide stabilizer is 1-hydroxyethylidene-1,1-diphosphonic acid or physiologically compatible salt thereof, said stabilizer being present from 0.005 % to 0.2 % by weight.

8. Use of a solution according to any of claims 1 to 7 as a preserved saline, as a preserved contact lens stabilizing solution, or as a preserved contact lens care solution for contact lenses during non-wearing periods.

## Patentansprüche

1. Konservierte, gepufferte, isotonische, salzhaltige Kontaktlinsenlösung, die mit der Augenumgebung verträglich ist und einen pH-Wert von 5,5 bis 8 aufweist, **dadurch gekennzeichnet, dass** die Lösung im Wesentlichen besteht aus
(i) Natriumperborat oder Natriumperoxid als Wasserstoffperoxidquelle zur Bereitstellung von Wasserstoffperoxid in einer Menge von 0,001% und mehr, jedoch weniger als 0,01% (100 ppm) auf das Gewicht,
(ii) einem Wasserstoffperoxidstabilisator, der eine Phosphonsäure darstellt, ausgewählt aus einer Verbindung der Formel (I) 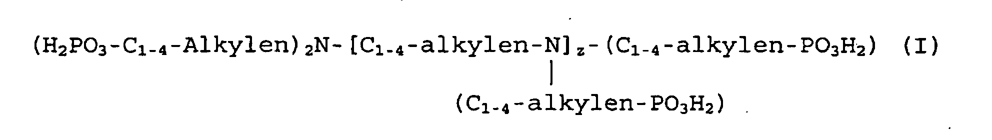 worin z eine ganze Zahl von 0 bis 3 ist und einer Verbindung der Formel (II) 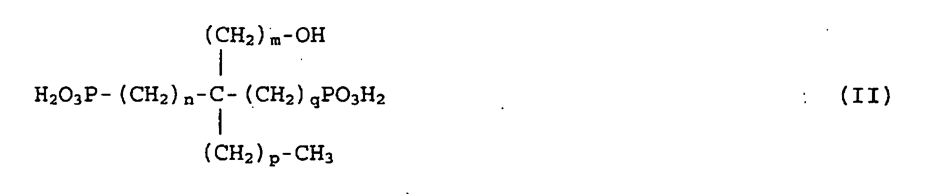 worin jeder von n, m, p und q unabhängig 0-4 ist und die physiologisch verträglichen Salze davon,
(iii) einem Puffer und
(iv) Wasser, Natriumchlorid und gegebenenfalls einem weiteren die Tonizität erhöhenden Mittel.

2. Lösung gemäß Anspruch 1, wobei die Lösung einen pH-Wert von 6,5 bis 7,5 aufweist.

3. Lösung nach Anspruch 1 oder 2, wobei Natriumperborat als eine Wasserstoffperoxidquelle verwendet wird.

4. Lösung nach Anspruch 3, wobei eine Natrium enthaltende Boratverbindung und Borsäure als Puffer (iii) verwendet werden.

5. Lösung nach einem der Ansprüche 1 bis 4, wobei der Wasserstoffperoxidstabilisator Diethylentriaminpenta(methylenphosphonsäure) oder ein physiologisch verträgliches Salz davon ist.

6. Lösung nach einem der Ansprüche 1 bis 5, die 0,002% bis 0,03% Wasserstoffperoxidstabilisator umfasst.

7. Lösung nach einem der Ansprüche 1 bis 4, wobei der Wasserstoffperoxidstabilisator 1-Hydroxyethyliden-1,1-diphosphonsäure oder ein physiologisch verträgliches Salz davon ist, wobei der Stabilisator mit 0,005% bis 0,2 Gewichtsprozent vorliegt.

8. Verwendung einer Lösung nach einem der Ansprüche 1 bis 7 als eine konservierte salzhaltige Lösung, als eine konservierte Kontaktlinsen-stabilisierende Lösung oder als eine konservierte Kontaktlinsenpflegelösung für Kontaktlinsen während der Zeiträume, in denen sie nicht getragen werden.

## Revendications

1. Solution saline pour lentilles de contact conservée, tamponnée, isotonique, qui est compatible avec l'environnement oculaire et a un pH de 5,5 à 8, **caractérisée en ce que** ladite solution est constituée essentiellement :
(i) de perborate de sodium ou de peroxyde de sodium comme source de peroxyde d'hydrogène, fournissant du peroxyde d'hydrogène en une quantité égale ou supérieure à 0,001 %, mais inférieure à 0,01 % (100 ppm) en poids,
(ii) un stabilisant du peroxyde d'hydrogène, qui est un acide phosphonique choisi entre :
un composé de formule (I) : 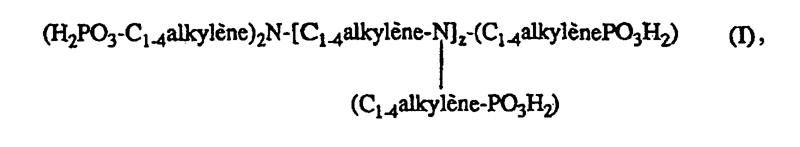 dans laquelle z est un nombre entier de 0 à 3 ; et
un composé de formule (II) : 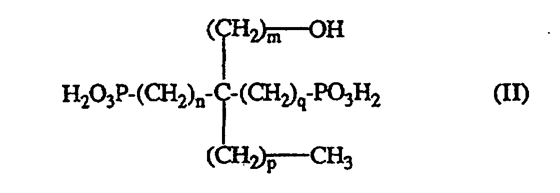
dans laquelle n, m, p et q ont chacun indépendamment une valeur de 0 à 4, et ses sels physiologiquement compatibles,
(iii) un tampon, et
(iv) de l'eau, du chlorure de sodium et facultativement un autre agent d'amélioration de la tonicité.

2. Solution selon la revendication 1, dans laquelle ladite solution a un pH compris entre 6,5 et 7,5.

3. Solution selon la revendication 1 ou 2, dans laquelle on utilise du perborate de sodium comme source de peroxyde d'hydrogène.

4. Solution selon la revendication 3, dans laquelle on utilise un composé boraté contenant du sodium et de l'acide borique comme tampon (iii).

5. Solution selon l'une quelconque des revendications 1 à 4, dans laquelle ledit stabilisant de peroxyde d'hydrogène est l'acide diéthylène-triaminepenta(méthylène)phosphonique, ou un de ses sels physiologiquement compatible.

6. Solution selon l'une quelconque des revendications 1 à 5, comprenant 0,002 % à 0,03 % d'un stabilisant de peroxyde d'hydrogène.

7. Solution selon l'une quelconque des revendications 1 à 4, dans laquelle ledit stabilisant de peroxyde d'hydrogène est l'acide 1-hydroxy-éthylidène-1,1-diphosphonique ou un de ses sels physiologiquement compatible, ledit stabilisant étant présent à raison de 0,005 % à 0,2 % en poids.

8. Utilisation d'une solution selon l'une quelconque des revendications 1 à 7, en tant que sérum physiologique conservé, comme solution stabilisante conservée pour lentilles de contact, ou comme solution de soin de lentilles de contact conservée pour des lentilles de contact pendant les périodes où elles ne sont pas utilisées.
